# EUROPEAN PATENT APPLICATION

(11) **EP 4 644 405 A1**
(43) Date of publication of application: **05.11.2025**
(21) Application number: 23912908.3
(22) Date of filing: 27.12.2023
(51) Int. Cl.: C07K 1/36, C07K 1/22, C07K 1/18, C07K 1/34, C12N 15/10

(54) **PURIFICATION SYSTEM FOR BIOLOGICAL MATERIALS COMPRISING SEGREGATED PURIFICATION SECTIONS**

(30) Priority: 27.12.2022 KR 20220185424
(71) Applicant: Samsung Biologics Co., Ltd., Incheon 21987 (KR)
(72) Inventor: CHO, Youngjin, Incheon 22003 (KR); KIM, Heejeong, Incheon 21986 (KR); JUNG, Jaewoong, Incheon 22002 (KR); YOO, Sungchul, Incheon 21986 (KR); HA, Junghwan, Incheon 22021 (KR); KIM, Yoojin, Incheon 21986 (KR); SEO, Sangwon, Incheon 22003 (KR)
(74) Representative: Mewburn Ellis LLP
(86) International application number: PCT/KR2023/021643
(87) International publication number: WO 2024/144234

(57) **Abstract**

The present invention relates to a purification system and a method of purifying biological materials using same, the purification system comprising: (a) a first purification section comprising one or more purification units; and (b) a second purification section comprising one or more purification units and atmospherically segregated from the first purification section by blocking airflow therebetween. According to the purification system of the present invention, the time required for product-to-product change over (PCO) can be dramatically reduced, thus having the surprising effect of maximizing the operation time of a purification facility.

## Description

### [Technical Field]

The present invention relates to a purification system for biological materials including segregated purification sections and a method of purifying biological materials.

### [Background Art]

Preparation of biological materials for use as pharmaceuticals should be carried out in facilities that meet standards, such as good manufacturing practice (GMP). In particular, after biological materials are produced through microbial or cell culture, a purification system is essential to improve the purity of the target materials and remove contaminants such as viruses.

A single manufacturing facility is usually used to manufacture one biological material and then another biological material, rather than being used to manufacture only one material since construction of manufacturing facilities, i.e., microbial or cell culture facilities and purification facilities, that meet the licensing standards is costly.

In particular, contract development and manufacturing organizations (CDMOs) or contract manufacturing organizations (CMOs) must continuously manufacture products containing various biological materials. In this case, it is highly preferable to minimize the cost of additional investment in microbial or cell culture facilities and purification facilities by maximizing the operation time of the manufacturing facilities, i.e., microbial or cell culture facilities and purification facilities, for example, by maximizing the annual operation time, in consideration of various economic advantages.

One of the factors that has the greatest impact on the operation time of these microbial or cell culture facilities and purification facilities is the time required for the product-to-product change over (PCO), which is the process of changing the production line, i.e., the microbial or cell culture facilities and purification facilities, from production of specific biological materials to production of other biological materials.

In particular, in the production of biological materials for use as pharmaceuticals, the culture facilities and purification facilities for the production of other biological materials essentially need replacement, cleaning and/or sterilization of each facility and each unit included therein in order to prevent cross-contamination of biological materials that will be manufactured later due to residual substances or viruses during the previous manufacturing process of the biological materials.

Therefore, by minimizing the time required for such PCO, the operation time of the manufacturing facility can be maximized, and ultimately, the manufacturing cost or facility investment cost can be reduced, which is economically advantageous.

Meanwhile, for PCO of conventional purification facilities, all of various units, such as chromatography units and filtration units, included in each purification facility after all purification processes are completed are replaced, washed, and/or sterilized. In this case, even though the purification units in the front part of the purification facility have already completed the function of purification, they are not operated at all until the purification in the purification units in the back part thereof is completed, thus taking a lot of time for PCO. It is impossible to reduce the time required for PCO and thus the annual operation time of the purification facility is reduced overall.

### [Disclosure]

Therefore, the present invention has been made in view of the above problems, and it is an object of the present invention to provide a purification system that is capable of maximizing the operation time of a purification facility by reducing the time required for PCO and a method of purifying biological materials using the purification system.

In accordance with one aspect of the present invention, the above and other objects can be accomplished by the provision of a purification system for biological materials including:
(a) a first purification section including one or more purification units; and
(b) a second purification section including one or more purification units and being atmospherically segregated by blocking the airflow from the first purification section.

In accordance with another aspect of the present invention, there is provided a method of purifying a biological material using a purification system including:
(a) a first purification section including one or more purification units; and
(b) a second purification section including one or more purification units and being atmospherically segregated by blocking the airflow from the first purification section.

### [Description of Drawings]

FIG. 1 illustrates the total purification time and potential PCO initiation time in a conventional purification system.
FIG. 2 illustrates PCO initiation time and the purification time for each purification section in the purification system of the present invention.
FIG. 3 illustrates the effect of an off-line column packing section on the reduction of the PCO time in the purification system of the present invention.
FIG. 4 illustrates the disposition and operation method of a washing room and a vestibule of washing room in the purification system of the present invention.
FIG. 5 illustrates an operation method of a heating ventilation and air conditioning (HVAC) system in the disposition and operation method of the washing room and the vestibule of the washing room in the purification system of the present invention.

### [Best Mode]

Unless defined otherwise, all technical and scientific terms used herein have the same meanings as appreciated by those skilled in the field to which the present invention pertains.

In general, the nomenclature used herein is well-known in the art and is ordinarily used.

In one aspect, the present invention provides a purification system for biological materials including:
(a) a first purification section including one or more purification units; and
(b) a second purification section including one or more purification units and being atmospherically segregated by blocking the airflow from the first purification section.

As used herein, the term "purification system" encompasses all devices necessary for purification processes including various purification units for purifying biological materials from a solution containing the biological materials in need of purification, and is used interchangeably with the term "purification facility".

As used herein, the term "first purification section" and "second purification section" each mean a separate section segregated spatially and atmospherically from the outside, are usually preferably rooms including one or more doors without being limited thereto, and are used interchangeably with "first purification room" and "second purification room", respectively.

In addition, the expression "the first purification section and the second purification section are atmospherically segregated" means that the first purification section and the second purification section are spatially segregated by a wall, ceiling, floor, or the like, and airflow between the two sections by the heating ventilation and air conditioning (HVAC) system is completely blocked so that there is no airflow between the two sections and the airflow between the two sections is not mixed at all.

However, the purification units included in the first purification section and the second purification section are connected through tubes or pipes that are completely isolated and segregated from the outside, and purification is performed when a solution containing a biological material to be purified flows through these tubes or pipes.

The purification system according to the present invention is capable of performing the replacement, washing and/or sterilization of the purification unit in the first purification section during the purification process in the second purification section after the purification process in the first purification section is completed, and of immediately starting the purification of a new biological material different from the biological material purified in the second purification section in the first purification section where the replacement, washing and/or sterilization of the unit is completed, thus remarkably reducing the time required for PCO and providing a remarkable effect of maximizing the operation time of the purification facility.

For example, when, in the separation and purification system including a total of three chromatography units (one affinity chromatography unit and two ion exchange chromatography units) and a virus filter unit, the affinity chromatography unit and the virus filter unit take 48 hours, and the first and second chromatography units take 32 hours, the total purification time is 80 hours.

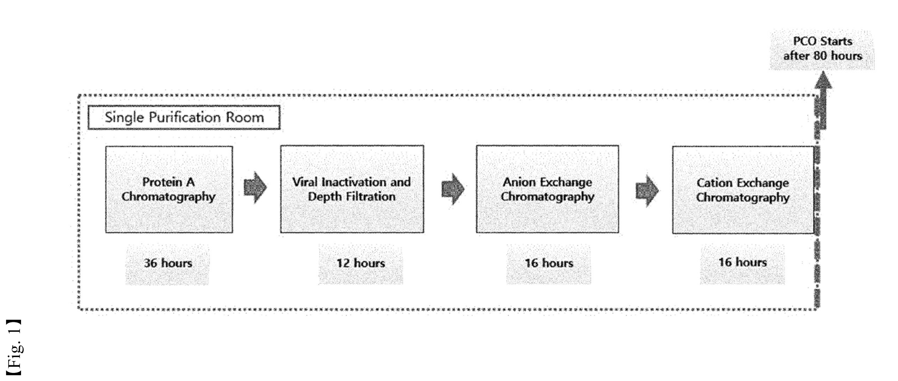

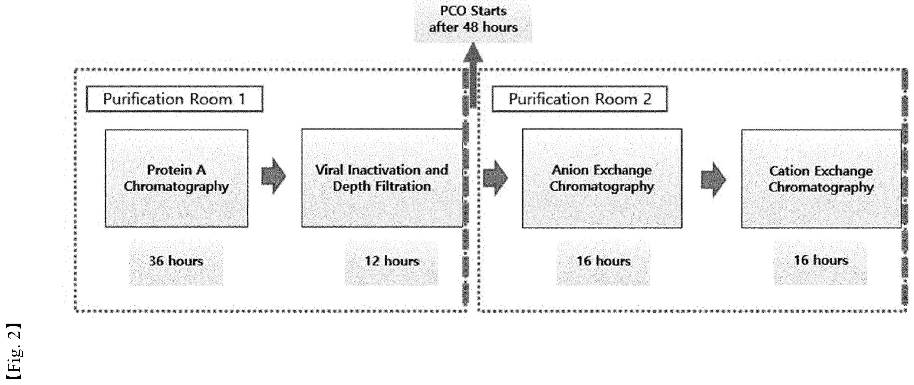

In a conventional purification system, PCO can only be started after all purification processes are completed, so that PCO can be started after 80 hours have passed from the time when the first purification is started. On the other hand, in the purification system according to the present invention including a first purification section and a second purification section atmospherically segregated from each other, PCO in the first purification section can be started even while the purification process in the second purification section is performed after the purification process in the first purification section is completed (i.e., after 48 hours have passed from the time when the first purification is started), so that the time required for the entire PCO can be significantly reduced (see FIGS. 1 and 2).

In the present invention, the purification unit included in the first purification section and the second purification section may be used without limitation as long as it is a purification unit or purification device commonly used in the art for purifying biological materials, and examples thereof include a chromatography unit, a filter unit, and a sterilization unit, and the purification unit may include at least one selected from the group consisting of the chromatography unit, the filter unit, and the sterilization unit, but is not limited thereto.

Non-limiting examples of the chromatography may include affinity chromatography, ion-exchange chromatography, hydrophobic interaction chromatography, size exclusion chromatography, and multi-mode chromatography, and the chromatography may include at least one selected from the group consisting of the exemplified chromatography, but is not limited thereto.

The affinity chromatography may be used without limitation as long as it uses a substance that can selectively and specifically bind to the substance to be purified. For example, immuno-affinity chromatography including a monoclonal or polyclonal antibody that specifically binds to the substance to be purified may be used, Protein A or Protein G, or the like may be used when the substance to be purified is an antibody, or Nickel-NTA, Avidin/Biotin, or the like may be used, but the embodiment is not limited thereto.

Ion exchange chromatography such as cation exchange chromatography or anion exchange chromatography may be used, but is not limited thereto.

In addition, any one may be used as the multi-mode chromatography without limitation as long as it has two or more characteristics, preferably two or more functions selected from affinity chromatography, ion exchange chromatography, hydrophobic reaction chromatography, and size exclusion chromatography.

Non-limiting examples of the filter unit may include a filter unit for microfiltration (MF), ultrafiltration (UF), nanofiltration (NF), depth filtration (DF), virus filtration, diafiltration, and ultrafiltration/diafiltration (UF/DF), and the filter unit may include at least one selected from the group consisting of these filter units, but is not limited thereto.

In addition, non-limiting examples of the sterilization unit may include a nanofiltration unit, a low-temperature sterilization unit, and a solvent/detergent treatment unit, and the sterilization unit may include at least one selected from the group consisting of these exemplified sterilization units, but is not limited thereto.

Preferably, the first purification section and the second purification section each include at least one chromatography unit as a purification unit and at least one purification section selected from the first purification section and the second purification section may further include at least one filter unit.

A ratio of the purification time (t₁) of the first purification section to the purification time (t₂) of the second purification section may be 1:0.5 to 1:1.5, preferably 1:0.8 to 1:1.2, more preferably 1:0.9 to 1:1.1, and most preferably 1:1. When the ratio of the purification time (t₁) of the first purification section to the purification time (t₂) of the second purification section falls within the range defined above, the time required for PCO may be reduced more efficiently.

The purification time (t₁) of the first purification section and the purification time (t₂) of the second purification section may be adjusted by controlling a combination of purification units included in each section.

For example, when one chromatography unit is included in the first purification section, the time required for purification is 24 hours, two chromatography units are included in the second purification section, and the total time required for purification is 36 hours, the ratio of the purification time (t₁) of the first purification section to the purification time (t₂) of the second purification section may be adjusted by introducing a filter unit that requires about 12 hours for purification, for example, a purification unit for ultrafiltration/diafiltration (UF/DF), in the first purification section without using an additional filter unit in the second purification section.

In one specific example, the purification unit for ultrafiltration/diafiltration (UF/DF) among the filter units is implemented in a movable form and can move between the first purification section and the second purification section, thus being used to adjust the ratio of the purification time (t₁) of the first purification section to the purification time (t₂) in the second purification section.

In addition, in the purification system according to the present invention, a flexible hose and/or hard piping may be used as a connecting means between the purification units. Preferably, it is advantageous to minimize the use of flexible hose and maximize the use of hard piping. As a non-limiting example, the ratio of hard pipes among the connecting means used in the entire purification system may be 50% or more, preferably 70% or more, more preferably 80% or more, and most preferably 90% or more, and the vessel size of each purification unit used herein may be adjusted if necessary.

The purification system according to the present invention may further include an off-line column packing section. The term "off-line column packing section" is used interchangeably with the term "off-line column packing room", and is preferably, but not limited to, spatially segregated from other sections, such as the first purification section and the second purification section, by a bulkhead, ceiling, and floor, and is completely blocked from air flow so that air between other sections is not mixed at all by an air conditioning system, or the like, and may include one or more inlets.

The off-line column packing section is a section where column packing and unpacking operations for column replacement in the chromatography unit in the PCO process are performed, and is preferably disposed adjacent to the first purification section and the second purification section, but is not limited thereto.

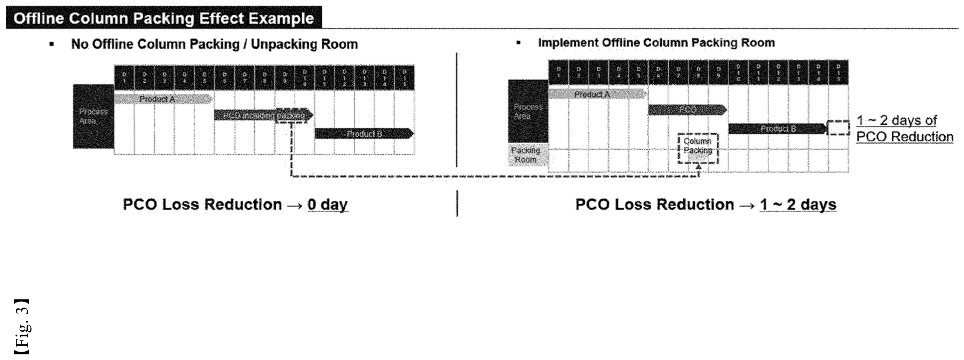

By separately providing off-line column packing sections as described above, the time required for column replacement in the chromatography unit on-site may be reduced and the overall time required for PCO may thus be reduced (see FIG. 3).

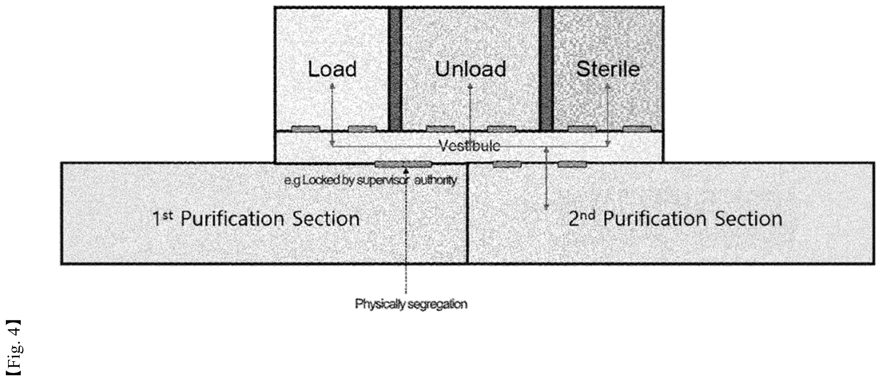

In addition, the purification system according to the present invention includes a washing room connected to the first purification section and the second purification section through a vestibule of a washing room, and it is preferable that the first purification section and the second purification section share the washing room (see FIG. 4).

As used herein, the expression "the first purification section and the second purification section share the washing room" means that washing of the purification units in the first purification section and the second purification section is performed in one washing room.

The vestibule of the washing room connecting the first and second purification sections to the washing room includes doors that allow access to both the first and second purification sections and the washing room, and the washing room may further include an autoclave room for sterilizing the washed purification units included in the first and second purification sections (see FIG. 4).

The doors that allow access to the first and second purification sections and the washing room in the vestibule of the washing room close while the normal purification process is in progress, and only essential personnel enter the necessary sections as necessary during PCO.

That is, after the purification process in the first purification section is completed, during the process of performing the purification process in the second purification section, while the purification unit, or the like in the first purification section is replaced, washed, and/or sterilized for PCO in the first purification section, the door connecting the vestibule of the washing room to the second purification section closes, and the door connected to the first purification section opens to perform PCO.

On the other hand, the purification unit or the like in the second purification section is replaced, washed, and/or sterilized for PCO in the second purification section during purification of a new biological material in the first purification section after the completion of PCO in the first purification section, the door connected to the first purification section closes and the door connected to the second purification section opens to perform PCO.

Preferably, the door connecting the vestibule of the washing room to the first purification section and the door connecting the vestibule of the washing room to the second purification section may be set not to open at the same time, thereby preventing the risk of cross contamination of the first purification section by viruses or other contaminants in the second purification section.

As a non-limiting example, the washing room may include one or more rooms selected from a loading room, a washing room, a drying room, and a sterilization room (see FIG. 4).

In particular, the first purification section, the second purification section, the washing room, and the vestibule of the washing room are preferably all normally atmospherically segregated except when the PCO process is performed, which can be achieved using a heating ventilation and air conditioning (HVAC) system.

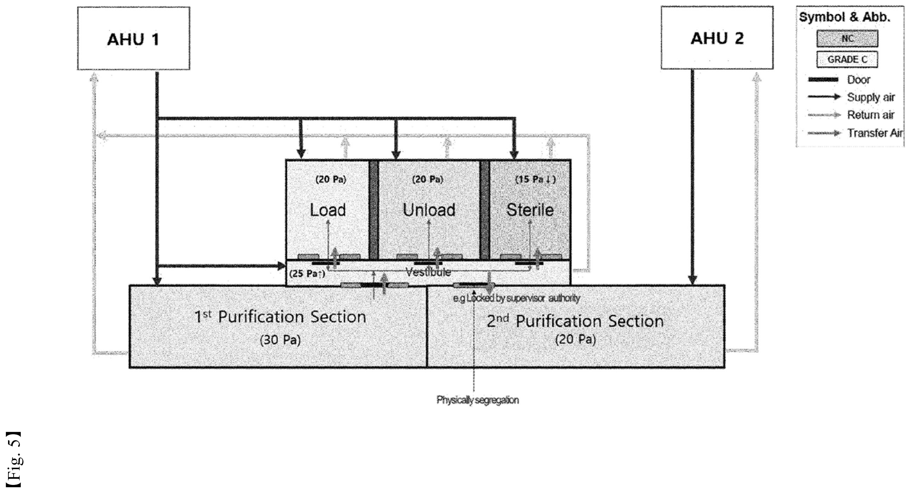

In addition, it is preferable that the pressures of the first purification section, the second purification section, the washing room, and the vestibule of the washing room be set differently so that the sections (rooms) have different pressures to prevent cross-contamination during the PCO process. Specifically, the air flow in the first and second purification sections has a one-way air flow direction from the first purification section to the second purification section, and thus the pressure of the first purification section should be set higher than the pressure of the vestibule of the washing room, the pressure of the vestibule of the washing room should be set higher than the pressure of the second purification section, and the pressure of the vestibule of the washing room should be set higher than the pressure of the washing room, and for example, each section may have a differential pressure of 5 Pa or more (see FIG. 5). By setting the pressure of the respective sections (rooms) differently in this way, the risk of cross-contamination of the first purification section by viruses or other contaminants in the second purification section may be prevented.

In another aspect, the present invention provides a method of purifying a biological material using a purification system according to the present invention, particularly a method of purifying a biological material with a reduced PCO time.

The method of purifying a biological material with a reduced PCO time according to the present invention includes:
(1) performing PCO in one or more purification units of a first purification section during or after purification of a first biological material in a second purification section after purification of the first biological material in the first purification section;
(2) introducing a solution containing a second biological material into the first purification section after the PCO in the first purification section to perform purification;
(3) performing PCO in the purification units in the second purification section after purification of the first biological material in the second purification section; and
(4) performing purification of a second biological material in the second purification section where the PCO is completed,
   but is not limited thereto.

The first biological material and the second biological material are preferably different biological materials, but even if they are the same biological material but manufactured in different batches, they are treated as different materials.

Examples of the biological material to be purified according to the present invention may include an antibody, mRNA, and protein medicine, but are not limited thereto.

The term "antibody", among the biological materials to be purified according to the present invention encompass any type of antibody including a monoclonal antibody, a polyclonal antibody, a bispecific antibody, and a multispecific antibody, and refers to a fragment of a monoclonal antibody and an antibody-drug conjugate (ADC).

The monoclonal antibody to be purified according to the present invention may be used without limitation as long as it specifically binds to an antigen related to a specific disease. Examples of the monoclonal antibody include, but are not limited to, Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab pegol, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Tegoprubart, Dapirolizumab pegol, I-131-Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Dinutuximab beta, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, CBP201, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab pegol, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab and the like.

In addition, examples of the bispecific/multispecific antibodies include Cinrebafusp alfa, Rozibafusp alfa, Obrindatamab, GEN1047, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Vobarilizumab, Cadonilimab, Vudalimab, Tebotelimab, Ivonescimab, Erfonrilimab, Bintrafusp alfa, Ozoralizumab, Faricimab, Vanucizumab, and Navicixizumab.

Examples of the antibody-drug conjugate include but are not limited to, naptumomab estafenatox, belantamab mafodotin, pivekimab sunirine, praluzatamab ravtansine, coltuximab ravtansine, denintuzumab mafodotin, loncastuximab tesirine, ibritumomab tiuxetan, inotuzumab ozogamicin, epratuzumab-cys-tesirine, moxetumomab pasudotox, Brentuximab vedotin, Gemtuzumab ozogamicin, Vadastuximab talirine, Lorvotuzumab mertansine, polatuzumab vedotin, tusamitamab ravtansine, telisotuzumab vedotin, rovalpituzumab tesirine, depatuxizumab mafodotin, farletuzumab ecteribulin, mirvetuximab soravtansine, trastuzumab deruxtecan, trastuzumab duocarmazine, trastuzumab emtansine, disitamab vedotin, pertuzumab zuvotolimod, patritumab deruxtecan, anetumab ravtansine, enfortumab vedotin, sacituzumab govitecan, and the like.

In addition, the protein medicine is a drug containing amino acids, has an effect of treating or preventing based on the activity of the protein, refers to a drug containing proteins other than antibody medicines, and may be selected from the group consisting of cytokines, therapeutic enzymes, hormones, soluble receptors and fusion proteins thereof, insulin or analogues thereof, EPO (erythropoietin), and serum- derived proteins, but is not limited thereto.

Among the biological materials to be purified according to the present invention, cytokines may be selected from the group consisting of interferon, interleukin, CSF (colony stimulating factor), TNF (tumor necrosis factor), and TGF (tissue growth factor), but are not limited thereto, and examples of the therapeutic enzyme may include beta-glucocerebrosidase (β) and agalsidase beta (agalsidase β), but are not limited thereto.

In addition, the soluble receptor refers to an extracellular domain of a receptor, and a fusion protein thereof refers to a protein in which an Fc region of an antibody is fused to the soluble receptor. The soluble receptor is a soluble form of a receptor to which a disease-related ligand binds, and examples thereof include, but are not limited to, a form in which an Fc region is fused to a TNF-α soluble receptor (for example, a product with the ingredient name "Etanercept" and a similar form), a form in which an Fc region is fused to a VEGF soluble receptor (e.g., a product with the ingredient name "Aflibercept" and a similar form), a form in which an Fc region is fused to CTLA-4 (e.g., a product with the ingredient name "Abatacept" or "Beladacept" and a similar form), a form in which an Fc region is fused to an interleukin-1 soluble receptor (e.g., a product with the ingredient name "Rilonacept" and a similar form), a form in which an Fc region is fused to an LFA3 soluble receptor (e.g., a product with the ingredient name "Alefacept" and a similar form) and the like.

The hormone contained in the pharmaceutical composition according to the present invention refers to a hormone or an analogue thereof that is injected externally for the treatment or prevention of a disease caused by hormone deficiency, or the like, and examples thereof include human growth hormone, estrogen, progesterone, and the like, but are not limited thereto.

The plasma-derived protein refers to a protein present in plasma, and refers to both those extracted from plasma and those produced recombinantly, and examples thereof include fibrinogen, von Willebrand factor, albumin, thrombin, FII (factor II), FV (factor V), FVII (factor VII), FVIII (factor VIII), FIX (factor IX), FX (factor X), and FXI (factor XI), but are not limited thereto.

In addition, mRNA is used as a mRNA vaccine and is used to express the desired protein in the body, similar to gene therapy.

Therefore, the mRNA to be purified according to the present invention may be mRNA encoding part or all of a virus, or mRNA encoding part or all of the protein drug, but is not limited thereto.

As used herein, the term "solution containing a biological material to be purified" includes any solution containing a material to be purified without limitation, and is preferably a culture solution of cells recombinant to express a biological material.

The cell may be a prokaryotic cell or a eukaryotic cell, the eukaryotic cell is preferably a mammalian cell, an insect cell, and a yeast, and the mammalian cell is preferably a murine myeloma (NSO) cell, a Chinese hamster ovary (CHO) cell, HT1080, HepG2, MCF7, Jurkat, PC12, BHK (baby hamster kidney cell), VERO, SP2/0, YB2/0, Y0, C127, L cell, COS, for example, COS1 and COS7, QC1-3, HEK-293, VERO, PER.C6, HeLA, or a hybridoma cell, and is more preferably a CHO cell, and examples of the CHO cell include a CHO-K1 cell, a CHO-K1 SV cell, a DG44 cell, a DUXB11 CHO cell, CHOS, a CHO GS knockout cell, a CHO FUT8 GS knockout cell, and the like, but is not limited thereto.

In addition, the yeast is preferably yeast of the genus *Pichia* or *Saccharomyces,* but is not limited thereto, and examples thereof include, but are not limited to, *Pichia pastoris, Pichia methanolica, Pichia kluyveri, Saccharomyces cerevisiae, Saccharomyces kluyveri, Saccharomyces uvarum,* and the like.

In addition, examples of the prokaryotic cell may include, but are not limited to, *Escherichia coli, Bacillus, Streptomyces, Streptococcus, Staphylococcus,* and *Lactobacillus.*

The cell culture solution is preferably a solution obtained by centrifuging the culture solution before applying the culture solution to the purification system according to the present invention and then performing depth filtration and sterile filtration of the supernatant, or a solution obtained by performing concentration and buffer exchange, but is not limited thereto.

Hereinafter, the present invention will be described in more detail with reference to examples. However, it is obvious to those skilled in the art that these examples are provided only for illustration of the present invention and should not be construed as limiting the scope of the present invention.

### [Example]

### Example 1. Time required for conventional biological material purification process in each purification unit

The main purification units of about 400 conventional biological material purification processes and the time required for purification for each purification unit were analyzed. As shown in Table 1, the result showed that most configurations including two to four chromatography units and the rest of units are for ultrafiltration/diafiltration (UF/DF) .

**[Table 1]**

| Purification time for biological material purification in each major unit | | | | | | |
|---|---|---|---|---|---|---|
| Process time (Hour) Study basis: (> 400 products avg.) | | Col 1 | Col 2 or UF/DF | Col 3 or UF/DF | Col 4 | Virus filtration |
| | | | * UF/DF is performed once | | | |
| % per Case | **Op. time** | **24** | **12** | **12** | **12** | **6** |
| 65% | Case 1 | 24 | 12 | 12 | - | 6 |
| | **Sub total** | **36** | | **18** | | |
| 30% | Case 2 | 24 | 12 (UF/DF) | 12 | 12 | 6 |
| | Sub total | **36** | | **30** | | |
| 5% | Case 3 | 24 | 12 | 12 (UF/DF) | 12 | 6 |
| | **Sub total** | **36** | | **30** | | |

| | | | | | | |
|---|---|---|---|---|---|---|
| (Col : Chromatography unit) | | | | | | |

### Example 2. Design of purification unit including separation section and reduction of PCO time

In the conventional Case 1 process described in Table 1, only the first chromatography, affinity chromatography, was included in the first purification section, and the remaining purification units, ion exchange chromatography and UF/DF, were included in the second purification section, so that the purification time (t₁) of the first purification section and the purification time (t₂) of the second purification section were set to 24 hours and 30 hours, respectively.

PCO was performed using the purification system according to the present invention having the segregated purification sections. The result showed that PCO could be successfully performed in accordance with the approval criteria, and the time required for PCO was also significantly shortened.

**[Table 2]**

| Design of purification unit having separation section according to the present invention | | | | | | |
|---|---|---|---|---|---|---|
| Process time (Hour) | | Col 1 | Col2 or UF/DF | Col3 or UF/DF | Col4 | Virus filtration |
| Conventional process | Case 1 | 24 | 12 | 12 | - | 6 |
| | **Sub total** | **36** | | **18** | | |
| Example 1 | Case 1 Replacement | First purification section | | Second purification section | | |
| | | 24 | - | 12 | 12 | 6 |
| | **Sub total** | **24** | | **30** | | |

Although the embodiments of the present invention have been disclosed for illustrative purposes, those skilled in the art will appreciate that various modifications, additions and substitutions are possible, without departing from the scope and spirit of the invention as disclosed in the accompanying claims.

Therefore, those skilled in the art will appreciate that this description is provided to set forth preferred embodiments for illustrative purposes and should not be construed as limiting the scope of the present invention. Therefore, the substantial scope of the present invention is defined by the accompanying filed claims and equivalents thereto.

### [Industrial Applicability]

The purification system according to the present invention is capable of performing replacement, washing and/or sterilization of the purification unit in the first purification section even during the purification process in the second purification section after the purification process in the first purification section is completed, and of immediately starting the purification of a new biological material different from the biological material purified in the second purification section in the first purification section where the replacement, washing and/or sterilization of the unit is completed, thus remarkably reducing the time required for PCO and providing a remarkable effect of maximizing the operation time of the purification facility.

## Claims

1. A purification system for biological materials comprising:
(a) a first purification section including one or more purification units; and
(b) a second purification section including one or more purification units and being atmospherically segregated by blocking the airflow from the first purification section.

2. The purification system according to claim 1, wherein the purification unit included in the first purification section and the second purification section comprise at least one selected from the group consisting of a chromatography unit, a filter unit, and a sterilization unit.

3. The purification system according to claim 2, wherein the chromatography unit comprises at least one selected from the group consisting of affinity chromatography, ion-exchange chromatography, hydrophobic interaction chromatography, size exclusion chromatography, and multi-mode chromatography.

4. The purification system according to claim 2, wherein the filter unit comprises at least one selected from the group consisting of filter units for microfiltration (MF), ultrafiltration (UF), nanofiltration (NF), depth filtration (DF), virus filtration, and ultrafiltration/diafiltration (UF/DF).

5. The purification system according to claim 2, wherein the sterilization unit comprises at least one selected from the group consisting of a nanofiltration unit, a low-temperature sterilization unit, and a solvent/detergent treatment unit.

6. The purification system according to claim 1, wherein the first purification section and the second purification section each comprise at least one chromatography unit as a purification unit, and at least one purification section selected from the first purification section and the second purification section further comprises at least one filter unit.

7. The purification system according to claim 1, wherein a ratio of a purification time (t₁) of the first purification section to a purification time (t₂) of the second purification section is 1:0.5 to 1:1.5.

8. The purification system according to claim 1, wherein the first purification section or the second purification section comprises a purification unit for ultrafiltration/diafiltration (UF/DF), and the purification unit for ultrafiltration/diafiltration (UF/DF) is implemented in a movable form and is movable between the first purification section and the second purification section.

9. The purification system according to claim 1, further comprising an off-line column packing section.

10. The purification system according to claim 1, wherein the purification system comprises a washing room connected to the purification section and the second purification section through a vestibule of a washing room.

11. The purification system according to claim 10, wherein the first purification section and the second purification section share the washing room.

12. The purification system according to claim 10 wherein the vestibule of the washing room connecting the first purification section and the second purification section to the washing room comprises a door that allows access to both the first purification section and the second purification section and the washing room.

13. The purification system according to claim 10, wherein the washing room further comprises an autoclave room for sterilizing of the purification unit included in the first purification section and the second purification section after washing.

14. The purification system according to claim 10, wherein the first purification section, the second purification section, the washing room, and the vestibule of the washing room are normally atmospherically segregated except when the PCO process is performed.

15. The purification system according to claim 14, wherein the atmospheric segregation of the first purification section, the second purification section, the washing room, and the vestibule of the washing room is performed through a heating ventilation and air conditioning (HVAC) system.

16. The purification system according to claim 10, wherein the pressures of the first purification section, the second purification section, the washing room, and the vestibule of the washing room are set to be different so that the pressures of the respective sections (rooms) are different to prevent cross-contamination during the PCO process.

17. The purification system according to claim 1, wherein the biological material is an antibody, mRNA, or a protein drug.

18. A method of purifying a biological material using the purification system according to any one of claims 1 to 17.

19. The method according to claim 18, comprising:
(1) performing PCO in one or more purification units of a first purification section during or after purification of a first biological material in a second purification section after purification of the first biological material in the first purification section;
(2) introducing a solution containing a second biological material into the first purification section after the PCO in the first purification section to perform purification;
(3) performing PCO in the purification units in the second purification section after purification of the first biological material in the second purification section; and
(4) performing purification of a second biological material in the second purification section where the PCO is completed.
